# EUROPEAN PATENT APPLICATION

(11) **EP 3 146 987 A1**
(43) Date of publication of application: **29.03.2017**
(21) Application number: 15002813.2
(22) Date of filing: 24.09.2015
(51) Int. Cl.: A61M 1/10

(54) **IMPELLER OF A HEART ASSISTING ROTARY BLOOD PUMP**

(71) Applicant: Rheinisch-Westfälische Technische Hochschule Aachen, 52062 Aachen (DE)
(72) Inventor: Böhning, Fiete, 52066 Aachen (DE); Groß-Hardt, Sascha, 52074 Aachen (DE); Steinseifer, Ulrich, 4730 Hauset (BE)
(74) Representative: Cohausz Hannig Borkowski Wißgott

(57) **Abstract**

The invention relates to an Impeller of a heart assisting rotary blood pump having a total number of blade passages (P) formed between adjacent blades (A, B, C,...) particularly the blade passages (P) being open or covered in axial direction, wherein the impeller comprises geometrically different blade passages (P1, P2, P3,...). The invention furthermore relates to a pump having such an impeller.

## Description

The invention relates to an impeller of a heart assisting rotary blood pump having a total number of blade passages formed between adjacent blades, particularly the blade passages being open or covered in axial direction. The invention furthermore relates to a rotary blood pump comprising such an impeller. Generally, the present invention also relates to the field of mechanical circulatory support devices, in particular the mentioned rotary blood pumps, for patients with heart failure.

Rotary blood pumps are often utilized as a ventricular assist device that is connected with the inlet to the native heart ventricle and with the outlet to the aorta thus bypassing in parallel the aortic heart valve.

It is known in the state of the art that an impeller of such centrifugal pump is formed of a disc having a circular cross section perpendicular to the axis of rotation, the disc supporting several blades for propelling the blood essentially in a radial direction. Or in the case of an axial flow blood pump, the impeller is formed of a shaft aligned to in the axis of rotation, supporting the blades for propelling the blood in axial direction or for the mixed flow pump, as combination of both. The invention, if applied to the mentioned kind of pump will provide the same construction of the impeller.

Between each pair of two blades a passage for the blood is formed that connects the inlet of the pump to the outlet. In a typical construction for a centrifugal pump the impeller has a channel being coaxial with the axis of rotation for feeding blood from the inlet to the passages.

In interaction with the native heart, such a pump is exposed to varying operating conditions with regard to the pressures at inlet and outlet of the pump. This variation comes from the contraction of the native ventricle, resulting in a high pressure during systole and a low pressure during diastole at the pump inlet.

Because of the aortic valve and the Windkessel effect, the pressure at the pump outlet is normally not exposed to such strong pressure fluctuations. Thus the pressure difference across the pump varies strongly with the frequency of the heartbeat. In rotary blood pumps, the relation of pressure difference across and flow through the pump is determined by the head capacity curve. In the case of a pressure fluctuation, the flow also fluctuates. The average flow of a native left ventricle can be 5 l/min, the average pressure rise per heartbeat cycle 100mmHg. Conditions of people having left ventricular failure can show lower average pressure rises and lower average flow.

In the design of rotary blood pumps, a specific operational point is often chosen for which the pump is optimized and thus the pump will have its best efficiency point at that condition. But when the pressure difference across the pump is varying, the pump will operate away from its best efficiency most of the time. The drawback of this situation is, that pumps that are designed in this manner will possess significantly reduced efficiencies for off design operating conditions.

The efficiency in a rotary pump is determined by the amount of losses it possesses. In general, hydraulic losses are caused by surface friction, eddy and separation losses due to changes in direction and magnitude of the velocity of flow. Geometrical features in a rotary pump that affect the efficiency are for example the size and distribution of cross section areas within the pump and in particular, the cross section area of the blade passage and the shape of the impeller blade.

The efficiency of such devices is important because the overall power consumption should be as low as possible. Pumps with high efficiency emit less heat, which is particularly important because the biological setting of blood pumps is very sensitive to increased temperatures, and can be operated for longer time on battery. Furthermore, high efficiency pumps can provide a desired hydraulic output at a lower rotational speed than a low efficiency pump. This is of importance as blood is very sensitive to elevated shear rates which depend on the rotational speed of the pump impeller.

It is an object of the invention to improve the construction of an impeller for such a blood pump to overcome the drawback of being optimized for just one operational point. It is furthermore an object of the invention to improve the overall efficiency of a pump.

According to the invention this object is solved by means of an impeller comprising geometrically different blade passages and by means of a pump having such an impeller.

By means of passages between the blades that have different geometry it is possible to optimize each one of the different passages to a different operational point of the pump during operation. Accordingly, the invention provides an impeller or pump that is designed not for one particular operating condition but for at least two, particularly multiple. The invention is applicable to any kind of pump type having an impeller, particularly the aforementioned types and preferably the centrifugal pumps.

Such a construction according to the invention results in a more even distribution of high efficiencies which allows the pump to operate more efficient over an operating range with fluctuating conditions instead of operation with best efficiency only at one specific operational point.

In a first embodiment of the invention it is possible that all blade passages are geometrically different thus providing an improved overall efficiency due to an optimization of each passage for another operational point of an operated pump.

In a second embodiment of the invention the total number of blade passages is formed by a set of at least two geometrically different blade passages that is positioned around the axis of rotation at least two times. Accordingly, in the circumferential direction of the impeller the mentioned set is repeated at least twice. With respect to the axis of rotation the repeated positioning of the mentioned set is preferably always the same but at different angles of rotation. Furthermore preferred the set is several times uniformly distributed around the axis of rotation meaning that the set is repeated at an angle of 360° / Number of repetitions with Number >=2.

In all the mentioned embodiments the different blade passages, particularly the different blade passages of the repeated set are optimized for different fluid-dynamical operating points, thus improving the efficiency of an operated pump over the entire operating range.

According to the invention, different geometrical construction of the passages, particularly of the ones of the repeated set, may be provided by means of different cross section areas of the blade passages, most preferred by means of different change of the cross section area in the radial direction, particularly if the invention is applied to centrifugal pumps or by means of different change of the cross section area in the axial direction, particularly if the invention is applied to axial pumps or by means of different change of the cross section area in both, axial and radial direction, particularly if the invention is applied to mixed flow pumps.

Such a change of the cross section area, particularly in radial or axial direction or combination of both directions, in a respective passage may be provided by a change in the respective radial or axial direction or both directions of the height of the passage or the height of adjacent blades above the blade ground and /or a change in the respective radial or axial or both directions direction of the width of the passage or the width of adjacent blades and /or different curvature of blade sidewalls.

In a preferred embodiment such changes may be nonlinear with the respective radial or axial position.

In another embodiment particularly of centrifugal pumps, that may be also combined with the aforementioned one, the blade passages, particularly the ones of the repeated set, may be geometrically different by means of different angles of adjacent blades at least at the inner and/or outer periphery of the impeller between a tangent to the respective blade sidewall and a tangent to the respective periphery.

These "inflow angles" or "outflow angles" also effect the efficiency of a respective passage and accordingly optimization of passages for different operational points may also be provided by such different angles.

The concept of the invention is furthermore described in view of the figures.

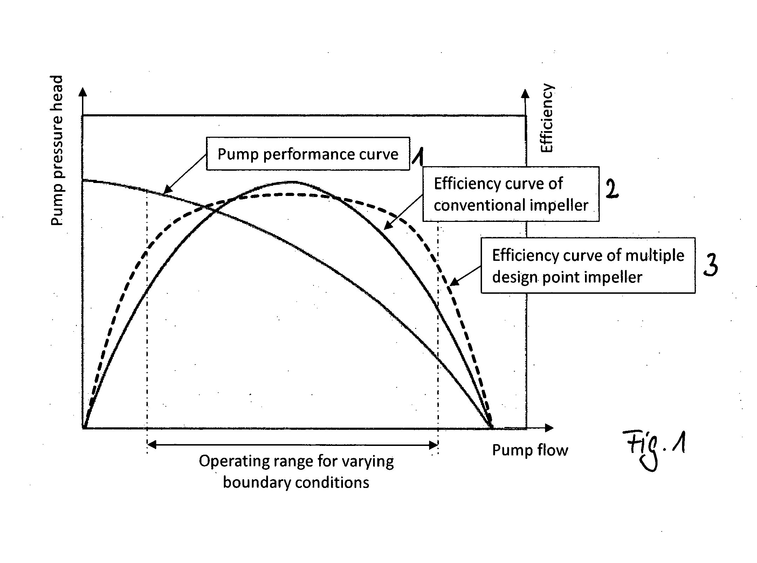

Figure 1 shows a pump performance curve 1 and pump efficiency curves 2, 3 for a conventional 1-point design 2 and the multiple-point design 3.

The invention in the design of the impeller is the geometrical feature of having multiple blades which each have a certain blade shape and cross-section area resulting in individual blade passages, whereby each one of the different blade passages is optimized for one specific operational point from multiple operational points which span over an operational range aside from an average operational point. Blade passages having the same geometry (shape) are optimized for the same operational point.

These multiple operational points can preferably be two, or more preferably three, or even four or more operational points. A set of at least two different passages is preferably symmetrically distributed at least two times along the impeller circumference.

It can be seen from figure 1 that curve 2 has a significant peak efficiency for a single operational point. In contrast to curve 2 the curve 3 is smaller in the absolute peak value but shows a less significant drop of efficiency on both sides of the maximum. Even though the maximum of curve 3 is smaller than the one of curve 2 the total area under curve 3 is bigger than the area under curve 1 proving a better overall efficiency for the entire operational range. The inventive design also provides less energy consumption compared to the conventional one since a pump will be operated the major part of the time (approx. 70%) in or near a diastole state, having higher efficiency in the inventive design at these operational points.

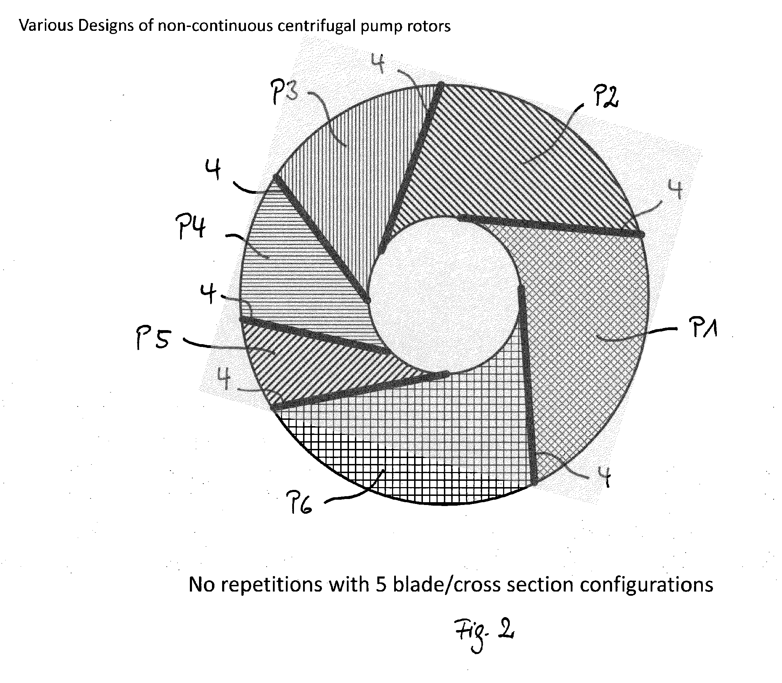

Figure 2 shows a first possible embodiment of the invention. The figure 2 illustrates an impeller of a centrifugal pump having a circular cross section in a plane perpendicular to the axis of rotation. The impeller furthermore has a central channel connecting the entrance areas of the respective passages to the inlet of the pump. This construction also applies to the embodiments described in the following figures. The blades 4 are just schematically shown. They may have the same geometrical shape but this is not mandatory. They may have a straight extension between the inner and outer periphery but may also be curved.

Figure 2 illustrates that the passages P1, P2, P3, P4, P5 and P6 all have different geometry which is preferably given by a different change of the cross section area of the passages in a direction from inner to outer periphery, particularly in radial direction. Accordingly this impeller may have passages each being optimized for a specific operational point within the entire operational range of a pump. Such impeller leads to curve 3 of figure 1 during operation in a pump. The passages P1, P2, P3, P4, P5 and P6 are each provided once on the impeller without repetition.

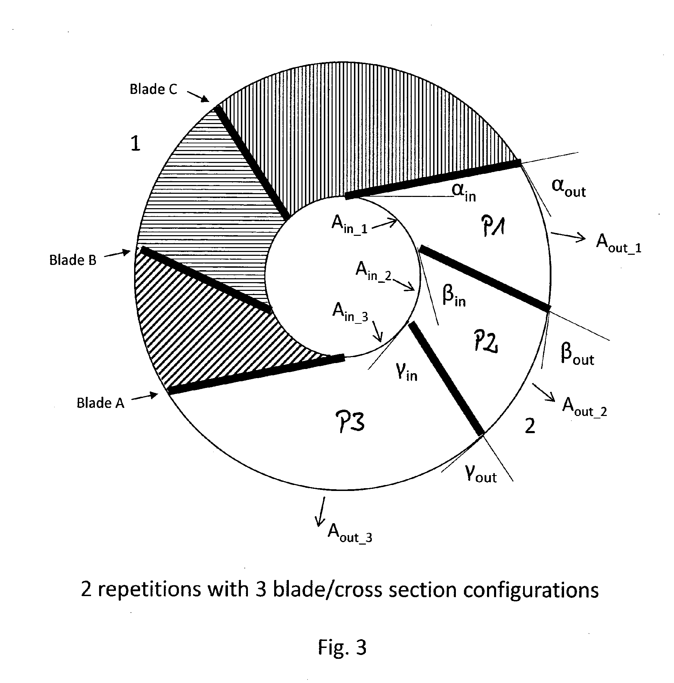

Figure 3 shows another example of an embodiment according to the invention. The impeller disc shown in top view supports six blades each pair of two blades surrounding a passage between the blades. As can be seen the total number of six blade passages is subdivided into a set of passages P1, P2, P3, each passage P1, P2 P3 having a different geometry and the set being repeated two times. Corresponding passages of the two times repeated set are spaced by 180°.

In this embodiment the impeller is optimized for three different operational points. The difference in geometry is here provided at least by different entrance angles αᵢₙ, βᵢₙ, λᵢₙ and exit angles αₒᵤₜ, βₒᵤₜ, λₒᵤₜ. Each angle is given between the tangent to the sidewall of a blade and the tangent to the inner / outer periphery of the impeller. The angle on the inner and outer periphery may be different.. The change from inner to outer blade angle can be of any steady form.

The difference in geometry may also be given by different change of the cross section area in radial direction, particularly the cross section being regarded as the cylindrical lateral surface inside each passage parallel to the axis of rotation..

The change of cross-section area within the blade passage of the blades can be achieved through change of blade height or through change of blade thickness, or both. The specific designs of the blade shapes of a set is given through the varying angles at the inner and out section of the blade. However, the specific design of the blade shape is not limited to only these angles.

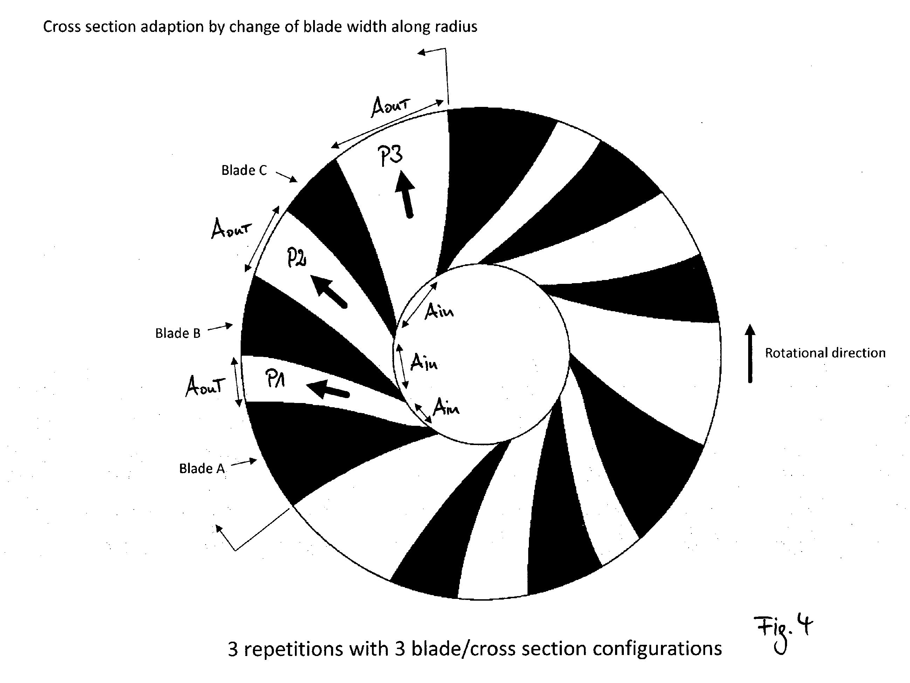

Figure 4 shows an embodiment of a set of passages P1, P2, P3 that is repeated three times along the periphery of the impeller or around the axis of rotation.

It can be seen here that the difference in geometry of the passages P1, P2, P3 may also be provided by different shapes of the blades A, B, C. These blades each have curved sidewalls facing the passage. Also the inner and outer cross section areas Aᵢₙ and Aₒᵤₜ of the respective passages are different. The change of cross-section area in between can be linear or follow another form of steady change.

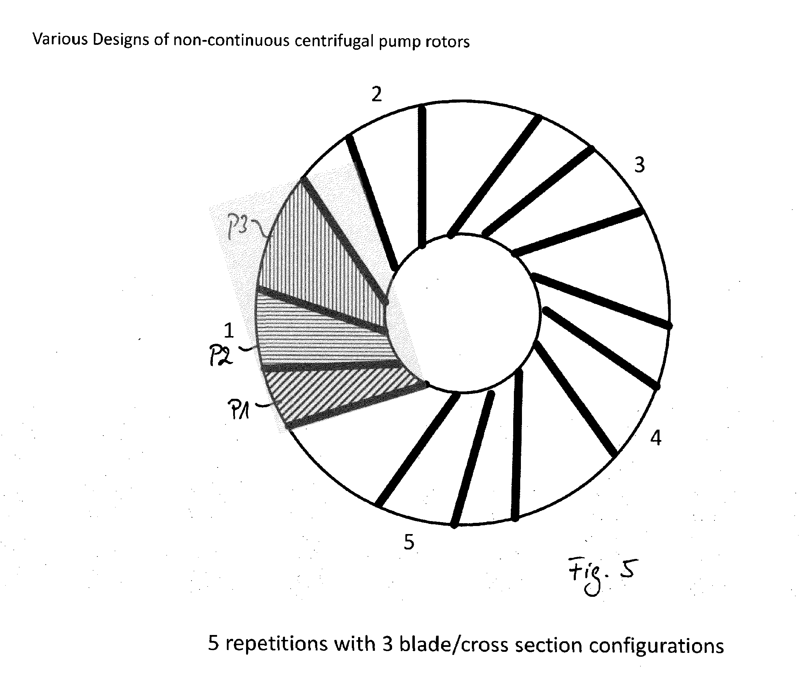

Figure 5 just shows a number of 5 repetitions of a set of 3 passages P1, P2, P3 on the impeller. The number is generally not limited. Again, each passage P1, P2, P3 of the set is optimized for a specific operational point. The blades are again just illustrated as straight lines but may be curved as shown in Figure 4 and may have also different shape.

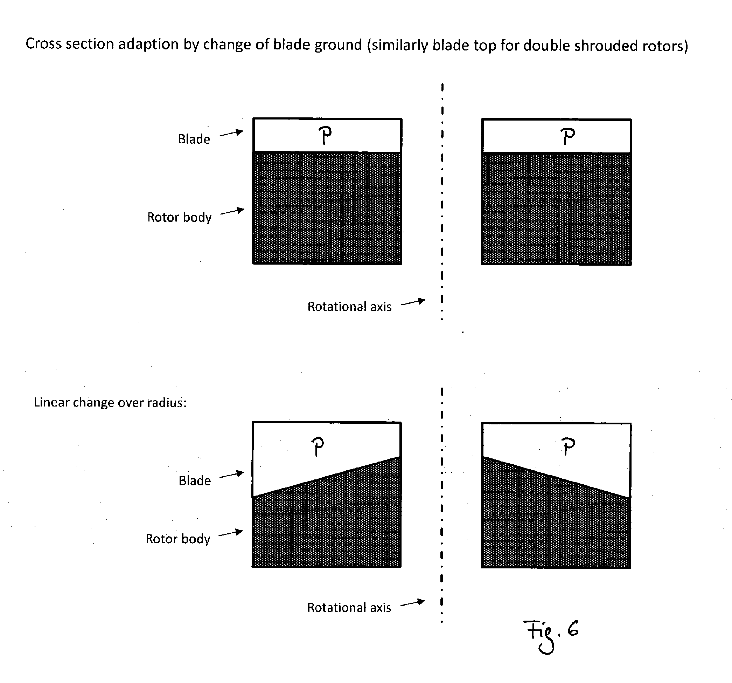

Figure 6 indicates cross sectional views of an impeller. The top view shows that the height of a passage P remains constant in radial direction. A change of the cross section area of a passage (cross section regarded perpendicular to radius and parallel to axis of rotation or regarded as the distribution of cylindrical lateral surface inside each passage parallel to the axis of rotation in radial direction) may be achieved by a change of the width of the passage regarded perpendicular to the radius and perpendicular to the axis of rotation.

The lower part shows that the height of a passage P may linearly increase with increasing radius what may be achieved by the fact that the thickness of the rotor body that supports the blades becomes thinner with increasing radius.

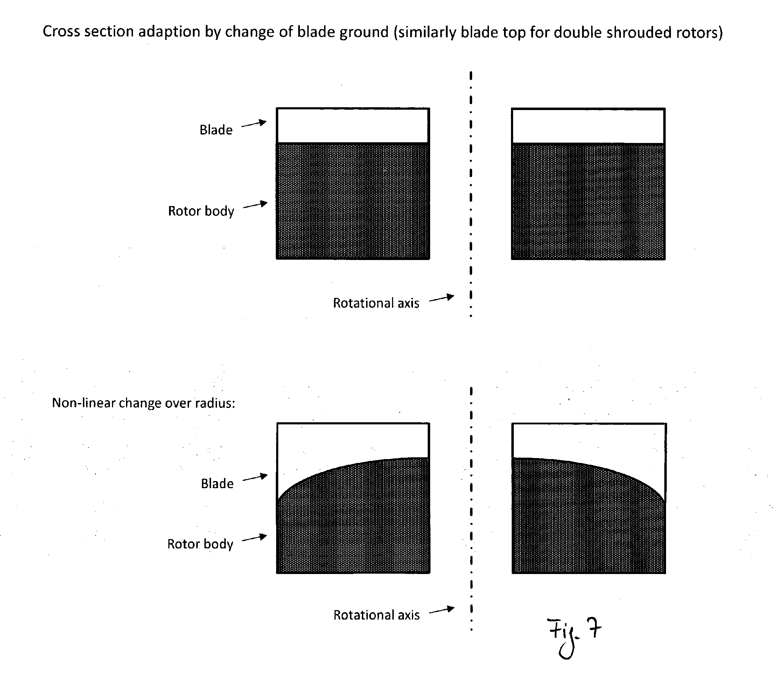

The bottom part of figure 7 shows an increase of the passage cross section area with increasing radius that is nonlinear.

In summary the invention provides the advantage of an impeller with multiple passages having particular geometrical feature that are optimal for a certain operational point. Each such passage may be repeatedly positioned on the impeller.

The geometrical features include blade shape, particularly the inner and outer blade angles, the cross-section area and the cross-section area distribution for the impeller blade and blade passage.

The advantage of the invention is to provide a broader high efficiency range and therefore better operation at fluctuating boundary conditions for instance given by a beating heart that is assisted with a pump.

Even though the figures describe the application of the invention in an impeller for a centrifugal pump it is apparent that the invention also applies for axial or mixed-flow pumps. Particularly for axial pumps all described features remain correct if a reference to radial direction is regarded as a reference to axial direction. Or particularly for mixed flow pumps, all described features remain correct if a reference to radial direction is regarded as a reference to both, radial and axial directions.

## Claims

1. Impeller of a heart assisting rotary blood pump having a total number of blade passages (P) formed between adjacent blades (A, B, C,...) particularly the blade passages (P) being open or covered in axial direction, **characterized in that** it comprises geometrically different blade passages (P1, P2, P3,...).

2. Impeller according to claim 1, **characterized in that** all blade passages (P1, P2, P3,...) are geometrically different.

3. Impeller according to claim 1, **characterized in that** the total number of blade passages (P) is formed by a set of at least two geometrically different blade passages (P1, P2, P3,...) that is positioned around the axis of rotation at least two times.

4. Impeller according to anyone of the preceding claims, **characterized in that** the different blade passages (P1, P2, P3,...), particularly the different blade passages (P1, P2, P3,...) of the repeated set are optimized for different fluid-dynamical operating points.

5. Impeller according to anyone of the preceding claims, **characterized in that** the different blade passages (P1, P2, P3,...), particularly of the repeated set, are geometrically different by means of different cross section areas of the blade passages (P1, P2, P3,...), particularly by means of different change of the cross section area, preferably in the radial or axial direction or both directions.

6. Impeller according to claim 5, **characterized in that** a change of the cross section area in the respective direction, is provided by
a. a change in the respective direction of the height of the passage (P1, P2, P3,...) or the height of adjacent blades (A, B, C...) above the blade ground and /or
b. a change in the respective direction of the width of the passage (P1, P2, P3,...) or the width of adjacent blades (A, B, C,...) and /or
c. different curvature of blade sidewalls.

7. Impeller according to claim 6, **characterized in that** the change is nonlinear with the respective position, particularly the radial position.

8. Impeller according to anyone of the preceding claims, **characterized in that** the different blade passages (P1, P2, P3,...), particularly of the repeated set, are geometrically different by means of different angles of adjacent blades (A, B, C,...) at least at the inner and/or outer periphery of the impeller between a tangent to the respective blade sidewall and a tangent to the respective periphery.

9. Impeller according to anyone of the preceding claims, **characterized in that** all blade passages (P) emerge from a common central channel passing through the impeller in axial direction or axial and radial direction.

10. Rotary blood pump comprising an impeller according to anyone of the preceding claims.
